# EUROPEAN PATENT APPLICATION

(11) **EP 2 177 629 A1**
(43) Date of publication of application: **21.04.2010**
(21) Application number: 08075890.7
(22) Date of filing: 19.11.2008
(51) Int. Cl.: C12Q 1/68

(54) **Shustring - multiple displacement amplification**

(30) Priority: 14.10.2008 EP 08075822
(71) Applicant: IPK Gatersleben Leibniz-Institut für Pflanzengenetik und Kulturpflanzenforschung, 06466 Gatersleben (DE)
(72) Inventor: Schmid, Karl, 70599 Stuttgart (DE)
(74) Representative: Boeckh, Tobias

(57) **Abstract**

The invention relates to a method for the amplification of a nucleic acid sequence. In particular the invention relates to a novel method suitable for large-scale amplification of nucleic acid sequences of any size, whereby the method does not require the whole sequence information of the target nucleic acid. The invention also relates to the use of this method, especially for analysis of clinical tissue samples, the determination of gene copy number variations, the obtainment of genetical markers for livestock and plant breeding or the detection of microorganisms. The invention also relates a set of primers and a kit.

## Description

### Summary

The invention relates to a method for the amplification of a nucleic acid sequence. In particular the invention relates to a novel method suitable for large-scale amplification of nucleic acid sequences of any size, whereby the method does not require the whole sequence information of the target nucleic acid. The invention also relates to the use of this method, especially for analysis of clinical tissue samples, the determination of gene copy number variations, the obtainment of genetical markers for livestock and plant breeding or the detection of microorganisms. The invention also relates a set of primers and a kit.

### Background

The amplification or enrichment of certain regions from complex genomes is a key step in many protocols for genome analysis. In the state of art two main amplification methods for targeted genomic regions are currently in use: (1) the cloning of large DNA fragments in genomic libraries and the subsequent screening and sequencing of gridded clones, and (2) the polymerase chain reaction (PCR) for the amplification of short genomic regions. Both methods have several drawbacks, especially in the use for large-scale amplification and analyses of genetic variation.

Clone-based genomic libraries are prohibitively expensive and require extensive screening, especially for species with large genomes. The establishment of genomic libraries is time-consuming and costly. So far it is not possible to automatize this method, which is why a high-throughput setup is not feasible. The creation of cloned libraries involves the preparation of high-molecular weight genomic DNA using an elaborated protocol. The DNA is then subjected to random physical shearing using ultrasound or other methods. Fragmented DNA is then size selected for fragments larger than 50 kb, or 100 kb, or 150 kb using gel-elektrophoresis, repaired at the ends to obtain DNA molecules with blunt end. To these DNA molecules, short doublestranded DNA linker molecules with defined restriction sizes are then added with DNA ligase, purified and ligated into a BAC or Cosmid vector. Competent E. Coli cells are then transformed with the circular ligation products. Thousands or ten thousands of transformants are randomly picked by robotors and gridded onto large cultivation plates. Colonies are transferred to glycerol stocks for long-term storage, and DNA is transferred to nylon membranes for later hybridization reactions to identify clones with desired inserts.

Current methods of PCR amplification involve the use of two primers which hybridize to the regions flanking a nucleic acid sequence of interest such that DNA replication initiated at the primers will replicate the nucleic acid sequence of interest. By separating the replicated strands from the template strand with a denaturation step, another round of replication using the same primers can lead to geometric amplification of the nucleic acid sequence of interest.
PCR amplification has the disadvantage that the amplification reaction cannot proceed continuously and must be carried out by subjecting the nucleic acid sample to multiple cycles in a series of reaction conditions. Although PCR is cheap and fast, it is only suitable in a high-throughput fashion for relatively short genomic regions of up to 10 kb.

Long range PCR is a modified PCR method, which allows the amplification of PCR products, larger than those achieved with conventional Taq polymerases. Up to 20 kb fragments are achievable from good quality genomic DNA. The method relies on a mixture of thermostable DNA polymerases, usually Taq DNA polymerase for high processivity (i.e. 5'-3' polymerase activity) and another DNA polymerase with 3'-5' proofreading abilities (usually Pwo). This combination of features allows longer primer extension than can be achieved with Taq alone. One of the disadvantages of this method is, that amplification of larger sequences like >20 kb is not possible.

Furthermore, in heterozygous or polyploid species, the resulting amplification products from PCR and long range PCR tend to be mixtures of different alleles or highly similar paralogous sequences, that are difficult to separate and to sequence. The determination of linkage phase for genetic mapping or differentiation of paralogs requires cloning and sequencing of individual fragments, which is expensive and error prone due to PCR-induced mutations and recombinations.

In response to these limitations, alternative methods have been developed that include selector technology, transformation-associated recombination (TAR) cloning and genomic selection using BAC clones or microarrays. These methods alleviate some of the problems of clone libraries and PCR, but they still have disadvantages such as lack of ability to provide high-throughput amplification, high setup costs or the requirement to know the entire sequence information for the region to be amplified. Therefore these methods are not suitable for the amplification of highly variable genomic regions.

So far it is not possible to amplify large targeted nucleic acid sequences fast and inexpensive in a high-throughput fashion, without having access to the whole sequence information.

### Summary of the invention

Accordingly, there is a need for the development of a method for the high-throughput amplification of multiple large genomic fragments that is cost effective, has little setup cost and does not require the complete sequence information of the target region.

The object of the invention was therefore to provide an amplification method for nucleic acid sequences, which fulfils these requirements and is superior to the state of the art.

### Description

Surprisingly the invention solves the above technical problem by providing a method comprising the following steps:
(a) providing the nucleic acid sequence;
(b) identification of binding sequences within the nucleic acid sequence, whereby the binding sequences are selected from the group comprising unique sequences, shortest unique sequences, conserved unique sequences, and/or repeated sequences;
(c) identification of primer sequences out of the identified binding sequences;
(d) amplification.

The invention relates to a method for the amplification of a nucleic acid of any size. The nucleic acid can originate from any nucleic acid containing object, cell, organism or parts thereof. A person skilled in the art knows how to isolate and prepare nucleic acid sequences for amplification. The invention is superior because the nucleic acid does not have to fulfil any necessary criteria.
The identification of binding sequences is an important step of the invention. The binding sequences of the invention can be identified with computer programs, which accelerate the whole process. It is preferred that the binding sequences are unique sequences among the target nucleic acid to prevent non-specific binding and therefore to enhance the specificity and the efficacy of the amplification process. The primer sequences of the invention are extracted from the set of all identified possible binding sequences. In a preferred embodiment of the invention, the primer sequences are complementary to the identified binding sequences. It is therefore preferred that the primers are unique sequences among the target nucleic acid. In another preferred embodiment the primer sequences are identified with a computer program, which simplifies the process. Anyhow a person skilled in the art is familiar with general criteria for the selection of primers, like for example minimum length or GC-composition. Therefore it is possible to exclude all the binding sequences that are not suitable as primer binding sequences by the use of general knowledge in the field of art. Using unique sequences as primers and/or primer binding sites prevents nonspecific priming and therefore leads to a better specificity and higher yield. Especially the use of the special primers of the invention allows the amplification of large sequences. Another advantage of the method is the fact that synthesis of primers can be carried out with standard methods.

Preferred is a large scale parallel synthesis of primers on a solid phase and/or subsequent release for amplification in a soluble phase. Therefore the invention provides an efficient and economical method for amplifying a nucleic acid sequence.

A preferred method of amplification is the multiple displacement amplification for linear nucleic acid sequences or the rolling circle amplification for circular nucleic acids. The combination of these amplification methods and the primers of the invention is surprisingly superior to the state of art in several aspects.
The method of the invention is superior because no expensive apparatuses are necessary. The only arising costs are expenses for primer design and reagents. Therefore the technical progress achieved by the invention reveals in a price-reduction, saving of time, material, work steps and costs. It was surprising that the method also shows an improved reliability and a better quality of the amplified nucleic acid compared to methods in the state of art.
It was especially surprising that the combination of the amplification method with the special primers of the invention makes large-scale or high-throughput amplification of sequences larger than 20 kb possible. Therefore the invention satisfies a long-felt need in the field of DNA amplification. Furthermore it was surprising that the method of the invention also increased the efficacy of the amplification and therefore led to a better yield.
Said advantages are shown especially in the preferential embodiments of the invention.
It was also surprising that the method can be carried out in the liquid phase with little setup costs, whereas commercially promoted methods rely on more expensive and elaborate solid phase enrichment by hybridization methods.
It was also surprising that the method can be carried out in the liquid phase with little setup costs, whereas commercially promoted methods rely on more expensive and elaborate solid phase enrichment by hybridization methods. It was not expected that the method allows the amplification of homologous, but highly variable genomic regions from closely related species.

The term "nucleic acids" as used herein may include any polymer or oligomer of pyrimidine and purine bases, preferably cytosine, thymine, and uracil, and adenine and guanine, respectively. See Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

In a preferred embodiment the identified primer binding sites are unique for the target nucleic acid. The use of unique primer binding sites prevents nonspecific priming, which is a disadvantage in the state of art. The achieved high specificity is benefiting because it leads to a higher efficacy and reduces the costs and the amount of work.
Especially preferred are shortest unique sequences. Using short unique primer is superior because the chances of mismatches are low. Also the costs and the necessary material are less compared to longer primers. One approach to identify a unique sequence is to search for all shortest unique sequences (shustrings) for a given nucleic acid sequence. In terms of the invention a local shustring is defined as the shortest sequence around a given base pair that occurs only once in the whole reference sequence (i.e., a genome).

Also preferred is the method, wherein the shortest unique substrings are identified by the use of bioinformatics programs. The use of such programs allows a fast and reliable identification of shustrings in a set of sequences. Especially preferred is the use of the program shustring version 2.2 due to its efficiency and reliability. This program is advantageous for the method of the invention because it reads the space efficient Fasta format. This is especially benefiting for the analyses of large sequences for example a whole genome. The use of Fasta format contributes to a fast and easy method of identifying shustrings. In the state of art some programs are known to generate an excessive number of files, which may break the file systems. The program shustring 2.2 is superior because these problems are avoided. The selection of primers is carried out by an additional program that analyses the shustring 2.2 output and searches for conserved sequencing using additional sequence data from other individuals of the same or from closely related species. Conserved primer sequences are then filtered for their suitability for the amplification step by choosing appropriately distanced primers with optimal sequence characteristics (GC-content, lack of repeats).

The term "target" as used herein refers to one or more nucleic acid regions of interest. Targets represent a subset of a genome or a nucleic acid sample. In general target regions will contain features of interest to be interrogated, for example, target regions may contain Single nucleotide polymorphisms (SNPs) or other polymorphisms, promoter regions, CpG islands, mutations, genes of interest, known or suspected regions of translocations and regions that are known to have copy number alterations that are associated with disease. The target regions are preferably amplified by the methods disclosed.

The term "polymorphism" as used herein refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms. SNPs are included in polymorphisms.

Another preferred embodiment of the invention is the identification of primer binding sites whose sequence is conserved in other individuals of the same species or in related species. The advantage of using conserved sequences is that the amplification can be rendered insensitive to sequence variation in the target region. Especially preferred are conserved sequences that occur only once in the whole reference sequence. The use of conserved unique sequences (CUS) is surprisingly benefiting in various fields like resequencing, cloning, genotyping or other types of analyses. One advantage is the use of conserved sequences from genetically divergent individuals of the same species or other species in the primer design step. This increases the likelihood that amplification works repeatedly and consistently because to some degree it is independent of sequence variation. Furthermore, since primer-binding sites are conserved in other species, they amplify homologous regions in closely relatives of the species from which the primers were designed. It was fully surprising that the use of CUS in combination with the amplification method also allows the amplification of large nucleic acid sequences in a high-throughput fashion.

Two nucleic aids sequences or parts thereof are substantially homologous to each other when the sequences exhibit at least about 40%-60%, preferably 60-70%, more preferably 70%-85%, more preferably at least about 85%-90%, more preferably at least about 90%-95%, and most preferably at least about 95%-98% sequence identity over a defined length of the molecules, or any percentage between the above-specified ranges, as determined using the methods above. As used herein, substantially homologous also refers to sequences showing complete identity to the specified nucleic acid sequence.

Also preferred is the method, wherein the CUS are identified by comparison of the identified shustrings to homologous regions in other individuals. These individuals can be of the same or another species. If the individual is of another species, related species are preferred. Especially preferred are homologous regions in other in other individuals, for which the whole sequence information is so far unknown.
It was surprising that the method of the invention can be used for the amplification of sequences and does not require the whole sequence information. This feature is especially advantageous, because it is possible to amplify unknown sequences easily and fast.
The use of CUS as primer binding sites in the method of the invention allows the amplification of sequences that are highly diverged between the primer binding sites and no a priori sequence information is necessary to amplify these regions. This characteristic is a major advantage of the method compared to the state of art. For example the microarray-based genomic selection of targeted region, where microarrays that contain sequences specific to a particular region are designed and hybridized against genomic DNA that was sheared to relatively small fragments of 1 kb. Any insertions that are larger than 1 kb and not present in the reference sequence will not be detected by the oligonucleotides on the array and are not available for subsequent analysis. This is a major limitation of the method, in particular for plant genomes, which can show high levels of small scale genomic rearrangments due to transposon insertions and other nonhomologous recombination. Surprisingly the method of the invention tolerates even rearrangements. In fact the method of the invention can be used to detect major rearrangements of particular regions by analysing a presence/absence pattern of amplification products. Thus the method of the invention, especially with CUS and/or conserved sequence overcomes these drawbacks and is therefore superior to the state of art. It was especially surprising that the method of the invention does not require the binding of all primers to homologous regions in other species.

In a preferred way to identify conserved unique sequences, it is necessary to first identify the shortest unique substring that begins with the focal base for every given base pair in the target region.

In another preferred embodiment of the invention, the repeated sequences are selected from the group comprising satellite DNA, minisatellite, microsatellite, short interspersed nuclear elements and/or long interspersed nuclear elements.

Satellite DNA consists of highly repetitive DNA. A repeated pattern can be between 1 base pair long (a mononucleotide repeat) to several thousand base pairs long, thus the total size of a satellite DNA block can consist of several megabases.

Satellite DNA is often localized to the telomeric or the centromeric region of a chromosome. The nucleotide sequence of the repeats is fairly well conserved across a species, which is why these regions can be used advantageously as conserved primer binding sequences.

In terms of the invention minisatellites consist of repetitive, variant repeats that range in length from 1 to over 100 nucleotides. These variant repeats can be tandemly intermingled and consist of at least 20% GC, preferred at least 40% GC, more preferred at least 50% GC. Preferred are minisatellites with more than 50% GC, more preferred more that 60%, especially preferred more than 70%. In terms of the invention minisatellites with more than 80% GC are also preferred, especially preferred are minisatellites with more than 90% GC. The use of minisatellites as primer binding sequences is surprisingly advantageous, especially for DNA fingerprinting analyses.

Microsatellites, or Simple Sequence Repeats, are polymorphic loci that consist of repeating units of 1 to 20 nucleotides in length. The use of microsatellites as primer binding regions is advantageous, especially because these sequences can be used as molecular markers. Microsatellites can also be used to study gene dosage, looking for duplications or deletions of a particular genetic region. Microsatellites can often be found in transcription units which can disrupt proper protein synthesis, leading to several diseases; for example myotonic dystrophy. Surprisingly the use of microsatellites in the method of the invention can be applied in diagnosis, follow-up treatment, treatment and aftercare of various diseases.

Short interspersed nuclear elements (SINE) are short DNA sequences (<1500 nucleotides). SINES are reverse-transcribed RNA molecules, which were originally transcribed by RNA polymerase III into tRNA, rRNA, or other small nuclear RNAs. SINEs seem to play a significant role in gene evolution, structure and transcription levels. The distribution of these elements has also been implicated in some genetic diseases and cancers. Therefore it is advantageous to use SINEs as primer binding sites in the method of the invention. Surprisingly SINEs can be used as primer binding sites in the analyses of various diseases.

Long interspersed nuclear elements (LINE) are long DNA sequences (>3kb). LINEs represent reverse-transcribed RNA molecules, which were originally transcribed by RNA polymerase II into mRNA. LINE elements can code for 2 proteins: (1) with the ability to bind single-stranded RNA, and (2) with reverse transcriptase and endonuclease activity, which enables them to copy both proteins and noncoding SINEs themselves. Surprisingly the use of LINEs as primer binding sites leads to superior results, especially in genetic fingerprinting.

The disclosed method can be used to clone or amplify any nucleic acid molecule of interest. The nucleic acid molecules can come from any source such as a cellular or tissue nucleic acid sample, a subclone of a previously cloned fragment, mRNA, chemically synthesized nucleic acid, genomic nucleic acid samples, nucleic acid molecules obtained from nucleic acid libraries, specific nucleic acid molecules, and mixtures of nucleic acid molecules. The disclosed method is particularly suited to producing libraries of cloned nucleic acid molecules starting with a complex mixture of nucleic acid molecules to be represented in the library. For example, cDNA can be produced from all of the mRNA in a cellular sample and used to make a cDNA library, or a library of genomic DNA can be produced from a genomic nucleic acid sample.

The target sequence can include multiple nucleic acid molecules, such as in the case of whole genome amplification, multiple sites in a nucleic acid molecule, or a single region of a nucleic acid molecule.

The term "genome" as used herein is all the genetic material in the chromosomes of an organism. DNA derived from the genetic material in the chromosomes of a particular organism is genomic DNA. A genomic library is a collection of clones made from a set of randomly generated overlapping DNA fragments representing the entire genome of an organism.

Also preferred is the method, wherein the nucleic acid sequence consists of more than 500 nucleotides and up to 1,000,000 nucleotides.

It was fully surprising, that the embodiments of the invention allow the amplification of large nucleic acid molecules, like » 20 kb. Even more surprising was the finding that with the method it is possible to amplify these large sequences in a high-throughput fashion, without forfeit the quality of the product.

Also preferred is the method, wherein the nucleic acid sequence is circular. The possibility of using circular targets is benefiting because lost of sequences of interest are circular, like bacterial plasmids or mitochondrial DNA. The use of such sequences leads to a high efficiency and a high yield. The circular nucleic sequence to be amplified can be any circular nucleic sequence of interest. Preferred are circular genomes or genomic regions. Circular genomes include, for example, organelle genomes, mitochondrial genomes, chloroplast genomes, plastid genomes, bacterial plasmid genomes, viral genomes, bacterial genomes, microbial genomes, and/or pathogen genomes or circularized parts thereof. Preferred are circularized genomic DNA, BAC clones or bacterial genomes or circularized parts thereof. The circular genome can be a naturally occurring genome, a genome that is not artificially modified, and/or a genome that is not an artificial nucleic acid or circularized parts thereof. The circular nucleic acid sequence can be double-stranded, single-stranded, or partially double-stranded. Circular nucleic acid sequence can occur in a variety of sizes and the disclosed methods can be used to amplify circular genomes of any size. For example, small viral genomes are typically between 5 and 40 kb, but some viral genomes are larger. Circular microbial genomes are known up to 1,500 kb. Accordingly, the circular genome to be amplified can have, for example, a length of from about 3000 to about 300000 nucleotides, about 4,000 to about 260,000 nucleotides, about 5,000 to about 150,000 nucleotides, or about 5,500 to about 40,000 nucleotides.

Also preferred is the method, wherein the nucleic acid sequence is linear. The amplification of linear nucleic sequence is surprisingly fast and very specific. The linear nucleic sequence to be amplified can be any linear nucleic sequence of interest. Preferred are genomes or genomic regions, especially preferred are eukaryotic chromosomes. The linear genome can be a naturally occurring genome, a genome that is not artificially modified, and/or a genome that is not an artificial nucleic acid or parts thereof. The linear nucleic acid sequence can be double-stranded, single-stranded, or partially double-stranded. Linear nucleic acid sequence can occur in a variety of sizes and the disclosed methods can be used to amplify linear genomes of any size. Accordingly, the linear genome to be amplified can have, for example, a length of from about 500 to about 1,000,000 nucleotides, about 4,000 to about 800,000 nucleotides, about 5,000 to about 500,000 nucleotides, about 10,000 to about 300,000 nucleotides, or about 50,000 to 150,000 nucleotides.

It was very surprising that the invention allows the use of the same setup for the amplification of linear and circular nucleic acids. This is advantageous because it simplifies the process and no special agents are necessary for either one of the targets.

Any material or sample containing or suspected of containing a nucleic acid of interest can be used as the nucleic acid sample. For example, the genomic nucleic acid sample can be a blood sample, a urine sample, a semen sample, a lymphatic fluid sample, a cerebrospinal fluid sample, amniotic fluid sample, a biopsy sample, a plant sample, a needle aspiration biopsy sample, a cancer sample, a tumour sample, a tissue sample, a cell sample, a cell lysate sample, a crude cell lysate sample, a forensic sample, an archeological sample, an infection sample, a nosocomial infection sample, an environmental sample, a soil sample, a water sample, a plant leaf sample, a pollen sample, a seed sample or combination thereof.

Primers for use in the disclosed amplification method are oligonucleotides having sequence complementary to the nucleic acid sequence. This sequence is referred to as the complementary portion of the primer. The complementary portion of a primer can be any length that supports specific and stable hybridization between the primer and the nucleic acid sequence. Generally this is 1 to 50 nucleotides long, but is preferably 6 to 30 nucleotides long, especially preferred 10 to 20 nucleotides.
A person skilled in the art knows which length is most suitable for a certain application. The hybridization between the primer and the target sequence is stronger when a long primer is used. The use of short primers has the advantage that melting temperature is similar to the amplification temperature. Therefore short primers have a high specificity and misspriming is prevented. Preferred are short primers without repeats and a balanced GC-content.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified. In many aspects of the present methods the primers are target specific primers that are capable of hybridizing specifically to a single location in a selected nucleic acid sequence of interest, for example, the human genome.

The term "oligonucleotide" or sometimes refer by "polynucleotide" as used herein refers to a nucleic acid ranging from at least 2, preferable at least 8, and more preferably at least 20 nucleotides in length or a compound that specifically hybridizes to a polynucleotide. Polynucleotides of the present invention include sequences of deoxyribonucleic acid (DNA) or ribonucleic acid (RNA) which may be isolated from natural sources, recombinantly produced or artificially synthesized and mimetics thereof. A further example of a polynucleotide of the present invention may be peptide nucleic acid (PNA). The invention also encompasses situations in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary.

The term "hybridization" as used herein refers to the process in which two single-stranded polynucleotides bind non-covalently to form a stable double-stranded polynucleotide; triple-stranded hybridization is also theoretically possible.

The primers can be composed in various ways. For example, the primers can each separately comprise deoxyribonucleotides, ribonucloetides, modified nucleotides, nucleotide analogs, labelled nucleotides, oligomer analogs, or a combination. In some forms of the disclosed methods and compositions, the primers can each contain at least one modified nucleotide such that the primers are nuclease resistant.

The primers can have any length that allows differential amplification of the nucleic acid of interest. The primers can have a complementary portion that can have any length that allows differential amplification of the nucleic acid of interest. For example, the primers can each separately have a length of, and/or can have a complementary portion having a length of, 5 nucleotides, 6 nucleotides, 7 nucleotides, 8 nucleotides, 9 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, or 30 nucleotides.

In another preferred embodiment of the invention primers consist of 1 to 50 nucleotides, preferred 6 to 30 nucleotides, especially preferred 10 to 20 nucleotides.

It is preferred that primers also contain additional sequence at the 5' end of the primer that is not complementary to the target sequence. This sequence is referred to as the non-complementary portion of the primer. The non-complementary portion of the primer, if present, serves to facilitate strand displacement during DNA replication. The non-complementary portion of the primer can also include a functional sequence such as a promoter for an RNA polymerase. The non-complementary portion of a primer may be any length, but is generally 1 to 100 nucleotides long, and preferably 4 to 8 nucleotides long.
It is also preferred that the non-complementary portion of the primer is a target for hybridisation in an enrichment step.
It is further preferred that the non-complementary portion of the primer is a restriction site which can be use in molecular cloning or circularization of the amplified molecules.

In a preferred embodiment of the invention, the primers contain phosphodiester bonds at the last 2 nucleotides. This is advantageous because the primers are protected from the exonuclease digestion by the Φ29 polymerase.

In another preferred embodiment, single strand binding protein can be added to the reaction. Surprisingly this enhances the specificity of the reaction. It was especially surprising that single strand binding protein can be used to increase the specificity of the reaction in species from which no prior sequence information is available. A high specificity is especially necessary in the use of the method in diagnoses of diseases for example cancer or for the identification of new markers, for example in plant breeding.

The set of primers can include any number of primers that allows differential amplification of the nucleic acid of interest. Generally, the number of primers can vary based on the size of the target nucleic acid. The number of primers can also vary based on whether the genome is single-stranded or double-stranded. The number of primers can also vary based on whether the genome is circular or linear. The number of primers can also vary based on the desired spacing between the primers when hybridized to the target nucleic acid. As an example, the set of primers can comprise 2 primers, 3 primers, 4 primers, 5 primers, 6 primers, 7 primers, 8 primers, 9 primers, 10 primers, 11 primers, 12 primers, 13 primers, 14 primers, 15 primers, 16 primers, 17 primers, 18 primers, 19 primers, 20 primers, 21 primers, 22 primers, 23 primers, 24 primers, 25 primers, 26 primers, 27 primers, 28 primers, 29 primers, 30 primers, 31 primers, 32 primers, 33 primers, 34 primers, 35 primers, 36 primers, 37 primers, 38 primers, 39 primers, 40 primers, 41 primers, 42 primers, 43 primers, 44 primers, 45 primers, 46 primers, 47 primers, 48 primers, 49 primers, 50 primers, 51 primers, 52 primers, 53 primers, 54 primers, 55 primers, 56 primers, 57 primers, 58 primers, 59 primers, 60 primers, 61 primers, 62 primers, 63 primers, 75 primers, 100 primers, 150 primers, 200 primers, 300 primers, or 400 primers.

In general, the more primers used, the greater the level of amplification that will be obtained. There is no fundamental upper limit to the number of primers that a set of primers can have. However, for a given target sequence, the number of primers in a set of primers will generally be limited to number of hybridization sites available in the target sequence.

In another preferred embodiment, the primer binding sequences have a GC-composition of 20-80%, preferred 30-70%, especially preferred 40-60%.

In another preferred embodiment, the primer sequences have a GC-composition of 20-80%, preferred 30-70%, especially preferred 40-60%.

Also preferred is the method with primers that have an alternating orientation among the binding sequences. Therefore the orientation of retained primer sequences is changed such that forward and reverse primers alternate with each other. This is especially advantageous because it maximises the chance that a strand displacement reaction starts during the amplification reaction.

It is preferred that the identified binding sequences for a given nucleic acid sequence are separated from each other. It is preferred that binding sequences are separated from each other by at least 5 bases. It is more preferred that the binding sequences are separated from each other by at least 100 bases. It is still more preferred that the binding sequences are separated from each other by at least 1,000 bases. It is still more preferred that the binding sequences are separated from each other by at least 2,000 bases. It is still more preferred that the binding sequences are separated from each other by at least 1,500 bases. It is still more preferred that the binding sequences are separated from each other by at least 3,000 bases. It is still more preferred that the binding sequences are separated from each other by at least 5,000 bases.

Also preferred is the method according to the invention with a spacing between the the binding sequences from 5 to 10,000 nucleotides, preferred 100 to 5,000 nucleotides, especially preferred 1,000 to 3,000 nucleotides.

It is preferred that the binding sequences are separated from each other by no more than about 10,000 bases. It is more preferred that the binding sequences are separated from each other by no more than about 8,000 bases. It is still more preferred that the binding sequences are separated from each other by no more than about 6,000 bases. It is still more preferred that the binding sequences are separated from each other by no more than about 5,000 bases. It is still more preferred that, the binding sequences are separated from each other by no more than about 4,000 bases. It is still more preferred that the binding sequences are separated from each other by no more than about 3,000 bases. Any combination of the preferred upper and lower limits of separation described above are specifically contemplated, including all intermediate ranges. The binding sequences need not to be separated from each other by the same number of bases. It is preferred that the binding sequences are separated from each other by about the same number of bases.

The optimal separation distance between binding sequences will not be the same for all DNA polymerases, because this parameter is dependent on the net polymerization rate. A processive DNA polymerase will have a characteristic polymerization rate which may range from 5 to 300 nucleotides per second, and may be influenced by the presence or absence of accessory single strand binding proteins and helicases. In the case of a non-processive polymerase, the net polymerization rate will depend on the enzyme concentration, because at higher concentrations there are more re-initiation events and thus the net polymerization rate will be increased. An example of a processive polymerase is Φ29 DNA polymerase, which proceeds at 50 nucleotides per second. An example of a non-processive polymerase is Vent exo(-) DNA polymerase, which will give effective polymerization rates of 4 nucleotides per second at low concentration, or 16 nucleotides per second at higher concentrations.

To obtain an optimal yield, the binding sequences spacing is preferably adjusted to suit the polymerase being used. Larger primer spacing distances are preferred when using a polymerase with a rapid polymerization rate. Shorter primer spacing is preferred when using a polymerase with a slower polymerization rate.

It is preferred that, when hybridized to the target sequence, the primers in a set of primers are separated from each other. It is preferred that, when hybridized, the primers in a set of primers are separated from each other by at least 5 bases. It is more preferred that, when hybridized, the primers in a set of primers are separated from each other by at least 100 bases. It is still more preferred that, when hybridized, the primers in a set of primers are separated from each other by at least 1,000 bases. It is still more preferred that, when hybridized, the primers in a set of primers are separated from each other by at least 2,000 bases. It is still more preferred that, when hybridized, the primers in a set of primers are separated from each other by at least 1,500 bases. It is still more preferred that, when hybridized, the primers in a set of primers are separated from each other by at least 3,000 bases. It is still more preferred that, when hybridized, the primers in a set of primers are separated from each other by at least 5,000 bases.

Also preferred is the method according to the invention with a spacing between the primers from 5 to 10,000 nucleotides, preferred 100 to 5,000 nucleotides, especially preferred 1,000 to 3,000 nucleotides.

It is preferred that, when hybridized, the primers in a set of primers are separated from each other by no more than about 10,000 bases. It is more preferred that, when hybridized, the primers in a set of primers are separated from each other by no more than about 8,000 bases. It is still more preferred that, when hybridized, the primers in a set of primers are separated from each other by no more than about 6,000 bases. It is still more preferred that, when hybridized, the primers in a set of primers are separated from each other by no more than about 5,000 bases. It is still more preferred that, when hybridized, the primers in a set of primers are separated from each other by no more than about 4,000 bases. It is still more preferred that, when hybridized, the primers in a set of primers are separated from each other by no more than about 3,000 bases. Any combination of the preferred upper and lower limits of separation described above are specifically contemplated, including all intermediate ranges. The primers in a set of primers need not, when hybridized, be separated from each other by the same number of bases. It is preferred that, when hybridized, the primers in a set of primers are separated from each other by about the same number of bases.

The optimal separation distance between primers will not be the same for all DNA polymerases, because this parameter is dependent on the net polymerization rate. A processive DNA polymerase will have a characteristic polymerization rate which may range from 5 to 300 nucleotides per second, and may be influenced by the presence or absence of accessory single strand binding proteins and helicases. In the case of a non-processive polymerase, the net polymerization rate will depend on the enzyme concentration, because at higher concentrations there are more re-initiation events and thus the net polymerization rate will be increased. An example of a processive polymerase is Φ29 DNA polymerase, which proceeds at 50 nucleotides per second. An example of a non-processive polymerase is Vent exo(-) DNA polymerase, which will give effective polymerization rates of 4 nucleotides per second at low concentration, or 16 nucleotides per second at higher concentrations.

To obtain an optimal yield, the primer spacing is preferably adjusted to suit the polymerase being used. Long primer spacing is preferred when using a polymerase with a rapid polymerization rate. Shorter primer spacing is preferred when using a polymerase with a slower polymerization rate.

In a preferred embodiment of the invention the amplification comprises bringing in contact a set of the identified primers, DNA polymerase and the nucleic acid sequence, where the nucleic acid sequence comprises a linear nuclei acid, and incubating the nucleic acid sequence under conditions that promote the replication of the nucleic acid sequence, wherein the replication of the nucleic acid sequence results in replicated strands, wherein during replication at least one of the replicated strands is displaced from the nucleic acid sequence by strand displacement replication of another replicated strand.
Preferred is the use of multiple displacement amplification

In another preferred embodiment the amplification comprises bringing into contact a set of primers, DNA polymerase, and a nucleic acid sequence, where the nucleic acid sequence comprises a circular nucleic acid, and incubating the nucleic acid sample under conditions that promote replication of the circular nucleic acid in the nucleic acid sample. Replication of the circular nucleic acid can proceed by rolling circle replication. The conditions that promote replication of the circular nucleic acid need not involve thermal cycling and/or can be substantially isothermic. Preferred is the use of rolling circle amplification

In another preferred embodiment, the amplification method is multiple displacement amplification (MDA) and/or rolling circle amplification (RCA). The use of these amplification methods is benefiting because no additional expensive apparatuses are necessary. It was surprising that the combination of the primes of the invention with MDA and/or RCA shows an improved reliability and a better quality of the amplified nucleic acid. It was nor expected that the use of MDA and/or RCA in the method of the invention leads to an increased efficacy and to a better yield. Especially surprising was the fact that combination of the method with the special primers of the invention allows high-throughput amplification of sequences larger than 20 kb, and is therefore superior to MDA or RCA in the state of art.

DNA polymerases useful in the method of the invention must be capable of displacing, either alone or in combination with a compatible strand displacement factor, a hybridized strand encountered during replication. Such polymerases are referred to herein as strand displacement DNA polymerases. It is preferred that a strand displacement DNA polymerase lack a 5' to 3' exonuclease activity. Strand displacement is necessary to result in synthesis of multiple copies of a target sequence. A 5' to 3' exonuclease activity, if present, might result in the destruction of a synthesized strand. It is also preferred that DNA polymerases for use in the disclosed method are highly processive. The suitability of a DNA polymerase for use in the disclosed method can be readily determined by assessing its ability to carry out strand displacement replication. Preferred strand displacement DNA polymerases are Φ29 DNA polymerase, Bst large fragment DNA polymerase, exo(-)Bca DNA polymerase, M2 DNA polymerase, ΦPRD1 DNA polymerase, Klenow fragment of DNA polymerase, T5 DNA polymerase, Sequenase and/or T4 DNA polymerase holoenzyme.

Especially preferred is the use of ΦM9 DNA polymerase. The use of Φ29 DNA polymerase together with conserved unique sequences as primer binding sites allows the amplification of sequences that are highly diverged between the primer binding sites and no a priori sequence information is necessary to amplify these regions. Surprisingly the use of Φ29 DNA polymerase in the method of the invention leads a highly accurate DNA synthesis and extremely high yields of amplified DNA even from minute amounts of target nucleic acids. The use of Φ29 polymerase is especially advantageous in combination with CUS or conserved sequences in other individuals or other species fragments, whereby the whole sequence of the target region is unknown. Surprisingly due to the use of Φ29 polymerase it is no requirement all primers bind to homologous regions in other species.

Strand displacement can be facilitated through the use of a strand displacement factor, such as helicase. It is considered that any DNA polymerase that can perform strand displacement replication in the presence of a strand displacement factor is suitable for use in the disclosed method, even if the DNA polymerase does not perform strand displacement replication in the absence of such a factor. Strand displacement factors useful in strand displacement replication include BMRF1 polymerase accessory subunit, adenovirus DNA-binding protein, herpes sirnplex viral protein ICP8, single-stranded DNA binding proteins, phage T4 gene 32 protein and calf thymus helicase. Especially preferred is the use of the single-stranded DNA binding proteins TthSSB-255. Surprisingly the addition of TthSSB-255 enhances the specificity of the reaction,

Also preferred is the method, wherein the conditions that promote replication of the nucleic acid sequence are substantially isothermic. Preferred is a temperature between 20° C and 45° C, especially preferred 25 - 40° C, exceptionally preferred 37°C.
The optimal temperature fort he method can vary according several factors, for example the length of the primers. A skilled person knows which factors can influence the optimal temperature for the reaction or is able to determine it within very few experiments.

In another preferred embodiment, the method is preformed as high-throughput. It was fully surprising that the method of the invention can be used for high-throughput and/or large-scale amplification without any loss of specificity or a decreased yield. So far there is no amplification method in the state of art suitable for high-throughput amplification of large (> 10 kb or > 20 kb) nucleic acid sequences. The method of the invention in a high-throughput fashion is reduces the amount of work, time and costs, furthermore there are no forfeits in quality and efficiency.
Especially preferred is the use of microtiterplates. Because only few components need to be combined in a small reaction volume, the automated pipetting of the reaction and execution in 96-well or 384-well microtiterplates can be easily performed using standard laboratory robots. Each well can contain DNA from different individuals that are combined with a mastermix containing the remaining reaction components. The isothermal amplification reaction and the subsequent purification of the reaction products can also be executed in the same process on a laboratory robot, which allows the processing large numbers of samples. This is a great advantage compared to hybridization-based enrichment methods with solid-phase primers.

Following amplification, the amplified sequences can be used for any purpose, such as uses known and established for amplified sequences. For example, amplified sequences can be detected using any of conventional detection systems for nucleic acids such as detection of fluorescent labels, enzyme-linked detection systems, antibody-mediated label detection, and detection of radioactive labels. A preferred form of labeling involves labeling of the replicated strands using terminal deoxynucleotidyl transferase. The replicated strands can be labeled by, for example, the addition of modified nucleotides, such as biotinylated nucleotides, fluorescent nucleotides, 5 methyl dCTP, BrdUTP, or 5-(3-aminoallyl)-2'-deoxyuridine 5'-triphosphates, to the 3' ends of the replicated strands. Amplification of forensic material for RFLP-based testing is one useful application for the disclosed method.

In a preferred embodiment of the invention the primers are labeled with a detection label. Labeled primers can be used to further manipulate or analyze amplified sequences and therefore are advantageous for several downstream applications. Especially preferred is the use of biotin, digoxigenin, FITC, rhodamin, AMCA, P-32, S-35 and/or H-3.

In another preferred embodiment of the invention, the amplified nucleic acid sequence is extracted with a molecule corresponding to the detection label. The advantage of this embodiment is especially an increased yield. Preferred is the use of streptavidin beads or anti-digoxigenin-antibodies.

In another preferred embodiment MDA and/or RCA is repeated with random hexamer primers.

In an especially preferred embodiment, the amplified nucleic acid sequence is used for downstream applications, preferred sequencing, cloning, digestion and subcloning for functional analysis and/or genotyping.

In another preferred embodiment the method is used for the amplification of nucleic acids from clinical tissue samples, preferred tumour tissue.
With this method it is possible to amplify mutations in the genome easily, which is important in the diagnosis, treatment, follow-up treatment or after care of various diseases. This method can be used in the diagnosis, treatment, follow-up treatment and after care of every disease associated with mutations. These mutations can occur in any the patient's cells, for example a cancer cell, or in a pathogen infecting a patient. A pathogen according to the invention is a microorganism, a virus, a bacterium, a fungi or a parasite.

In another preferred embodiment the method is used for determination of gene copy number variations. Thereby it is possible to distinguish between healthy and diseased tissue. This is an important feature in the diagnosis or therapy of various diseases. In cancer cells gene copy number are often elevated. For instance, the EGFR copy number can be higher than normal in cell lung cancer. It is also known that a low copy number of FCGR3B can increase susceptibility to Systemic Lupus Erythematosus and similar inflammatory immune system disorders. Copy number variation has also been associated with autism, schizophrenia, and idiopathic learning disability. These are only a few examples that show what an important role the gene copy number play in certain diseases. Therefore an easy method to determine and compare gene copy numbers is benefiting for the diagnosis, treatment, follow up treatment and after care of all diseases that are associated with an altered gene copy number.

The method of the invention is also preferred because it can be used in sequencing cancer genes from tissue biopsies. Sequencing of cancer cells revealed a plethora of mutations contributing to the transition of cells into a state of constant proliferation. In response to this observation, the cancer genome project has been devised which is aimed at characterizing the most common mutations that are associated with cancer genes, which can then subsequently used either for diagnosis or the development of treatments. The method of the invention is uniquely suited to amplify complete cancer genes, because mutations likely involved in the formation of cancer are not restricted to the relatively short coding regions of oncogenes but also to regulatory regions and sequence motifs affecting splicing. In this context the use of conserved unique sequences is especially advantageous because mutations or rearrangements can be detected easily with this method.

In another preferred embodiment the method is used to obtain genetical markers for livestock and plant breeding.

Animal and plant breeders are interested in markers that are specific for various traits and are polymorphic in the germplasm they are utilizing in their breeding work. By sequencing their lines in candidate regions after using a marker obtained with a method of the invention, they are able to identify novel markers available for them.

In another preferred embodiment the method is used for the detection of microorganisms. Shortest unique substrings that are only present in a pathogen microorganism, but not in closely related non-pathogen strains are chosen. By doing so it is possible to distinguish between both groups -pathogen and non-pathogen microorganisms-.

Also preferred is the use of the method for sequence analyses of large genomic regions. This is important in the biomedical field as well as in the filed of livestock and plant breeding.

In a preferred embodiment of the invention, the method is used to manufacture primers.
Primers and any other oligonucleotides can be synthesized using established oligonucleotide synthesis methods. Methods to produce or synthesize oligonucleotides are well known in the art. Such methods can range from standard enzymatic digestion followed by nucleotide fragment isolation to purely synthetic methods, for example, by the cyanoethyl phosphoramidite method. Solid phase chemical synthesis of DNA fragments is routinely performed using protected nucleoside cyanoethyl phosphoramidites.
So long as their relevant function is maintained, primers, detection probes, address probes, and any other oligonucleotides can be made up of or include modified nucleotides (nucleotide analogs). Many modified nucleotides are known and can be used in oligonucleotides. A nucleotide analog is a nucleotide which contains some type of modification to the base, sugar or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Certain nucleotide analogs, such as 5-substituted pyrimidines, 6-azapyrimidines and N-2, N-6 and O-6 substituted purines, including 2-aminopropyladenine, 5-propynyluracil, 5-propynylcytosine and 5-methylcytosine can increase the stability of duplex formation. Other modified bases are those that function as universal bases. Universal bases include 3-nitropyrrole and 5-nitroindole. Universal bases substitute for the normal bases but have no bias in base pairing. That is, universal bases can base pair with any other base. Base modifications often can be combined with for example a sugar modification, such as 2'-O-methoxyethyl, to achieve unique properties such as increased duplex stability.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C1 to C10, alkyl or C2 to C10 alkenyl and alkynyl. 2' sugar modifications also include but are not limited to-O[(CH₂)n O]m CH₃, --O(CH₂)n OCH₃. --O(₂)n NH.sub.2, --O(₂)n CH₃, --O(CH₂)n-ONH₂, and --O(CH₂)nON[(CH₂)n CH₃)]₂, where n and m are from 1 to about 10. Other modifications at the 2' position include but are not limited to: C1 to C10 lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂, CH₃, ONO₂, NO₂ N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included.

It is understood that nucleotide analogs need only contain a single modification, but may also contain multiple modifications within one of the moieties or between different moieties.

Oligonucleotides can be comprised of nucleotides and can be made up of different types of nucleotides or the same type of nucleotides. For example, one or more of the nucleotides in an oligonucleotide can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. Such oligonucleotides can be referred to as chimeric oligonucleotides.

The invention also relates to a set of primers, obtained by a method of the invention.

The invention also relates to a kit comprising a set of primers obtained by the method of the invention.
In an especially preferred embodiment the kit comprises in addition to the set of primers, the DNA polymerase, dNTPs, yeast pyrophosphatase and/or reaction Buffer, optionally together with information on how to combine the contents of the kit.

Kit components in a given kit can be designed and adapted for use together in the disclosed method. For example, disclosed are kits for amplifying nucleic acid sequences, the kit comprising at least a reaction buffer and a DNA polymerase. The components of such a kit are described elsewhere herein. In some forms of the disclosed kits, the kit can further comprise a set of primers. In some forms of the disclosed kits, the kit can further comprise a lysis solution. In some forms of the disclosed kits, the kit can further comprise a stabilization solution. In some forms of the disclosed kits, the kit can further comprise deoxynucleotide triphosphates. In some forms of the disclosed kits, the kit can further comprise one or more detection probes. Detection probes are described elsewhere herein. In some forms of the kit, the detection probes can each comprise a complementary portion, where the complementary portion is complementary to a nucleic acid sequence of interest. In some forms of the kit, the kit can further comprise denaturing solution. In some forms of the kit, the kit can further comprise reaction mix. The kits also can contain nucleotides, buffers, detection probes, fluorescent change probes, lysis solutions, stabilization solutions, denaturation solutions, or a combination.

Any of the components that can be present in a kit that can be used together can be combined in a single component of the kit. Thus, a reaction mix can include, for example, buffers, deoxynucleotide triphosphates and primers. Similarly, components and solutions can be divided into constituent parts or sub-solutions. The kits can be used for any purpose, generally for nucleic acid amplification. In some forms, the kit can be designed to detect nucleic acid sequences of interest in a genome or other nucleic acid sample. In some forms, the kit can be designed to assess a disease, condition or predisposition of an individual based on a nucleic acid sequence of interest. In other forms, the kit can be designed to assess genetical markers, preferred for livestock and plant breeding.

The invention also relates to a pharmaceutical composition, comprising the primers obtained by a method of the invention. Preferably this pharmaceutical composition is used in diagnosis of a disease. Especially preferred is the use in the diagnosis of cancer.

### Examples

### Example 1:

Identification of shortest unique substrings in the tropinone-reductase like gene cluster of Arabidopsis thaliana
The growing availability of completely sequenced genomes greatly facilitates the identification of conserved unique sequences. Using the completely sequenced genome of Arabidopsis thaliana, we identified local shortest unique substrings for the region spanning the tropinone reductase-like gene family. This gene family consists of 13 genes that are located on chromosome 2, and is one of the largest tandemly repeated gene families in the A. thaliana genome. Comparative sequencing of the TRL cluster in closely related species such as Arabidopsis lyrata, Arabidopsis cebennensis, Capsella rubella, and Boechera drummondii indicated that the region is rapidly evolving both in terms of sequence divergence in noncoding regions, and also in copy number variation. After identifying local shustrings from the A. thaliana sequence with the shortest unique substrings 2.2 program on chromosome 2, filtering for conserved shortest unique substrings was achieved comparisons of shustrings to homeologous BAC sequence from A. lyrata. Among the remaining CUS, 20 primers with a length of 12 or 13 nucleotides were selected; they cover a region of 79,280 base pairs with an average spacing of 3.96 kb. The TRL region primer sequences are available as supplementary information.

### Example 2:

### Specificity of amplification

The annealing temperature of 12- and 13-mer primers is about 40°C on average and higher than the temperature used for the isothermal amplification. Therefore, a certain level of unspecific annealing and amplification is expected when genomic DNA is used as a template. On the other hand, the strand displacement activity of the Φ29 polymerase enables the annealing of neighboring primers to newly synthesized DNA; this branching process leads to an exponential amplification of the target region because only for this region primers have other matching primers that will initiate the branching.

### Example 3:

### Amplification from other species

One advantage of CUS-MDA is the use of conserved sequences from genetically divergent individuals of the same species or closely related other species in the primer design step. This increases the likelihood that CUS-MDA amplification works repeatedly and consistently because to some degree it is independent of sequence variation. Furthermore, since primer-binding sites are conserved in other species, they are expected to amplify homologous regions in closely relatives of the species from which the primers were designed. Due to the high processivity of the Φ29 polymerase, fragments > 20 kb are synthesized by the enzyme and it is no requirement all primers bind to homologous regions in other species. However, the less primer binding sites are conserved the fewer DNA is synthesized in a CUS-MDA reaction. To test this application we used the TRL primers to amplify the TRL region from several close relatives of A. thaliana. These species include A. arenosa, Boechera drummondii, Capsella rubella, Olimarabidopsis pumila and Cleome spinosa.

### Example 4:

### Effect of single stranded protein on amplification specificity

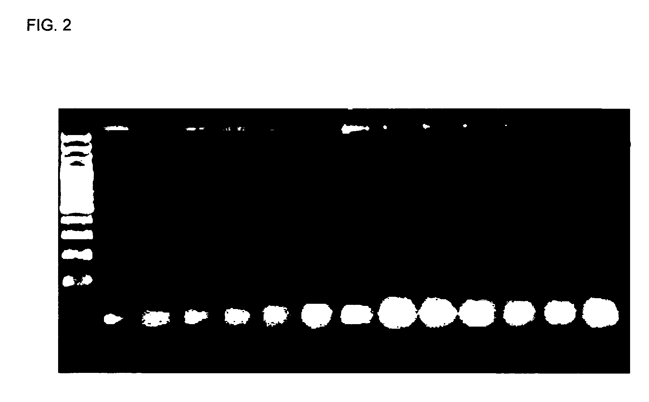

It has been shown that the specificity of the MDA reaction can be increased by the addition of single stranded protein (SSB), which stabilizes single-stranded DNA. To increase the specificity of the reaction, we added TthSSB-255 single stranded protein created by Inoue et al. (2006) to the reaction (Figure 2). While there is only little improvement in the amplification of the A. lyrata sample, which was used to design the primers, there is a marked improvement in the A. cebennensis DNA, even with a very small amount of the protein. This confirms earlier results on the effect of this protein, and shows that SSB can be used to increase the specificity of the reaction in species from which no prior sequence information is available.

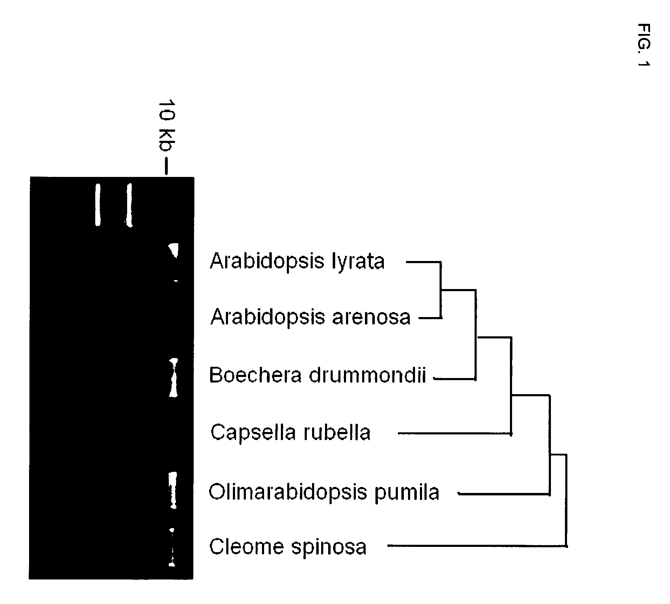

Figure 1 shows that the amplification in different species, even distantly related ones such as Cleome spinosa works with the CUS primers. Figure 1 furthermore shows amplification of the TRL region with CUS-MDA from other species related to Arabidopsis thaliana. Amplifications were conducted with 50 ng genomic DNA from each species. The phylogenetic relationships of the species is shown on top of the gel.

### Example 5:

### Effect of single stranded protein on amplification specificity

It has been shown that the specificity of the MDA reaction can be increased by the addition of single stranded protein (SSB), which stabilizes single-stranded DNA. To increase the specificity of the reaction, we added TthSSB-255 single stranded protein to the reaction (Figure 2). While there is only little improvement in the amplification of the A. lyrata samples, which were used to design the primers, there is a marked improvement in the A. cebennensis DNA, even with a very small amount of the protein. This confirms earlier results on the effect of this protein, and shows that SSB can be used to increase the specificity of the reaction in species from which no prior sequence information is available.

Figure 2 shows the effect of TthSSB-255 single-strand binding protein on amplification specificity. Different amounts of SSB protein were added to the MDA amplification of the TRL region in A. lyrata and A. cebennensis. Two µl of the reaction are shown on the gel.

### Example 6:

### Applications of CUS-MDA

Numerous applications of targeted amplification of homologous regions are possible with the CUS-MDA method. We demonstrate the application of this method by designing primer sets and amplification, and subsequent sequencing of the resulting products.

### Example 7:

### Allelic variation at a Mendelian disease locus

Cystic fibrosis is an autosomal recessive disease caused by mutations at the CFTR locus (OMIM:*602421). More than one thousand mutations at this locus are known to cause the disease, but commercial diagnosis tests have been developed for a core of several dozen mutations. Since the CFTR gene is very long (189 kb), mutations can not be identified easily by PCR amplification and sequencing. We developed CUS-MDA primers that amplify the whole region and make the sequence variation accessible for downstream applications such as rapid next-generation resequencing. Only few nanograms are necessary for the amplification reaction, therefore the method is suitable for both testing parents as carriers and subsequent analysis of embryo cells.

### Example 8:

### HLA typing

The MHC locus is one of the most polymorphic and rapidly evolving regions in the human genome. Various theories have been proposed that attempt to explain the diversity at this locus and it has been found that allelic variation at the HLA locus affects several types of human diseases including cancer, autoimmune diseases and graft rejection. The genotyping of HLA alleles is therefore an important component in the diagnosis and treatment of certain diseases. Comparative sequencing revealed the structure and the complex mechanisms of evolution at this locus that include gene duplication, gene conversion and natural selection of sequence diversity. CUS-MDA is able amplify the complete HLA region or several loci within the complex for further sequencing and haplotype/allele determination.

### Example 9:

### Sequencing of cancer genes from tissue biopsies

Sequencing of cancer cells revealed a plethora of mutations contributing to the transition of cells into a state of constant proliferation. In response to this observation, the cancer genome project has been devised which is aimed at characterizing the most common mutations that are associated with cancer genes, which can then subsequently used either for diagnosis or the development of treatments. CUS-MDA is uniquely suited to amplify complete cancer genes, because mutations likely involved in the formation of cancer are not restricted to the relatively short coding regions of onkogenes but also to regulatory regions and sequence motifs affecting splicing.

### Example 10:

### Resequencing of association study loci

Association studies identify candidate genomic regions that may harbor alleles causing diseases or which are associated in favorable phenotypic variation for animal and plant breeding. CUS-MDA is uniquely suited to amplify the complete genomic region around significant markers and fine map the causative genes. Marker identification in animal and plant breeding Animal and plant breeders are interested in markers that are specific for various traits and are polymorphic in the germplasm they are utilizing in their breeding work. By sequencing their lines in candidate regions after using a CUS-MDA marker, they are able to identify novel markers available for them.

## Claims

1. A method of amplifying a nucleic acid sequence, the method comprising:
(a) providing the nucleic acid sequence;
(b) identification of binding sequences within the nucleic acid sequence, whereby the binding sequences are selected from the group comprising unique sequences, shortest unique sequences, conserved unique sequences, shortest conserved unique sequences and/or repeated sequences;
(c) identification of primer sequences out of the identified binding sequences;
(d) amplification.

2. A method of amplifying a nucleic acid sequence preferred according to claim 1, the method comprising:
(a) providing the nucleic acid sequence;
(b) identification of primer sequences by means of the identification of binding sequences within the nucleic acid sequence, whereby the binding sequences are selected from the group comprising shortest unique sequences, conserved unique sequences and/or repeated sequences;
(c) bringing in contact a set of the identified primers, DNA polymerase and the nucleic acid sequence and incubating the nucleic acid sequence under conditions that promote the replication of the nucleic acid sequence, wherein the replication of the nucleic acid sequence results in replicated strands, wherein during replication at least one of the replicated strands is displaced from the nucleic acid sequence by strand displacement replication of another replicated strand, or
bringing in contact a set of the identified primers, DNA polymerase and the nucleic acid sequence, wherein the nucleic acid is circular and incubating the nucleic acid sequence under conditions that promote the replication of the circular nucleic acid sequence, wherein the replication of the circular nucleic acid sequence proceeds by rolling circle replication.

3. Method according to claim 1 or 2, wherein the nucleic acid sequence is circular and/orlinear and consists of more than 1000 nucleotides and up to 1,000,000 nucleotides.

4. Method according to any of the preceding claims, wherein the repeated sequences are selected from the group comprising satellite DNA, minisatellite, microsatellite, short interspersed elements and/or long interspersed elements.

5. Method according to any of the preceding claims, wherein the conserved unique sequences are identified by comparison of the shortest unique sequences to homologous regions in a related species.

6. Method according to any of the preceding claims, wherein the primers consist of 1 to 50 nucleotides, preferred 6 to 30 nucleotides, especially preferred 10 to 20 nucleotides.

7. Method according to any of the preceding claims, wherein the primers have an alternating orientation among the binding sequences.

8. Method according to any of the preceding claims, with a spacing between the primers from 5 to 10,000 nucleotides, preferred 100 to 5,000 nucleotides, especially preferred 1,000 to 3,000 nucleotides.

9. Method according to any of the preceding claims, wherein the amplification method is multiple displacement amplification and/or rolling circle amplification.

10. Method according to any of the preceding claims, wherein the DNA polymerase is Φ29 DNA polymerase.

11. Method to any of the preceding claims, wherein the conditions that promote replication of the nucleic acid sequence are substantially isothermic.

12. Method according to any of the preceding claims, wherein the primers are labeled with a detection label, selected from the group comprising biotin, digoxigenin, FITC, rhodamin, AMCA, P-32, S-35 and/or H-3.

13. Method according to any of the preceding claims, wherein amplified nucleic acid sequence is extracted with a molecule corresponding to the detection probe, preferred streptavidin beads and/or anti-digoxigenin-antibodies and MDA and/or RCA is repeated with at least one hexamer primer.

14. Method according to any of the preceding claims, wherein the amplified nucleic acid sequence is used for downstream applications, preferred sequencing, cloning, digestion and subcloning for functional analysis and/or genotyping.

15. Use of the method according to any of the preceding claims for
(a) amplification of nucleic acid sequences from clinical tissue samples, preferred
tumour tissue
(b) amplification of mutations in the genome;
(c) determination of gene copy number variations;
(d) obtainment of genetical markers for livestock and plant breeding;
(e) detection of microorganisms.

16. Use of the method according to any of the preceding claims to manufacture primers.
